Europäisches Patentamt

European Patent Office

Office européen des brevets

(11) Veröffentlichungsnummer: **0 375 991 B1**

(12) **EUROPÄISCHE PATENTSCHRIFT**

(45) Veröffentlichungstag der Patentschrift: **26.01.94**

(51) Int. Cl.5: **C08G 59/50**, C07D 233/16

(21) Anmeldenummer: **89122240.8**

(22) Anmeldetag: **02.12.89**

(54) **Verfahren zur Herstellung von faserverstärkten Wickelkörpern aus Epoxidharzen und [1(2'Aminoethyl)-1,3-diazacyclo-penten-2-yl-2]-heptan-2 und/oder [1(2'Aminoethyl)-1,3-diaza-cyclopenten-2-yl-2)]-2,4,4-trimethylpentan-1.**

(30) Priorität: **27.12.88 DE 3843986**

(43) Veröffentlichungstag der Anmeldung:
**04.07.90 Patentblatt 90/27**

(45) Bekanntmachung des Hinweises auf die Patenterteilung:
**26.01.94 Patentblatt 94/04**

(84) Benannte Vertragsstaaten:
**AT BE CH DE ES FR GB IT LI NL SE**

(56) Entgegenhaltungen:
**DE-A- 2 018 954**

**DATABASE CHEMICAL ABSTRACTS, (HOST:STN), Nr. 87(06): 040275w, 1977, Columbus, Ohio, US; & JP-A-7 726 598**

(73) Patentinhaber: **Witco GmbH**
**Ernst-Schering-Strasse 14,**
**Postfach 1620**
**D-59180 Bergkamen(DE)**

(72) Erfinder: **Burba, Christian, Dr.-Dipl.-Chem.**
**Gerhart-Hauptmann-Strasse 9**
**D-4715 Herbern(DE)**
Erfinder: **Mrotzek, Werner, Dr.-Dipl.-Ing.**
**Dresdener Strasse 24**
**D-4600 Dortmund 1(DE)**
Erfinder: **Franz, Herbert**
**Albert-Schweitzer-Strasse 41**
**D-4700 Hamm 1(DE)**
Erfinder: **Krotzek, Alwin**
**Nathstrasse 17**
**D-4712 Werne(DE)**

## Beschreibung

Die Erfindung betrifft ein Verfahren zur Herstellung von faserverstärkten Wickelkörpern, insbesondere Rohrhilfsstücken, durch Tränken temperaturbeständiger Fasern mit Bindemitteln auf Basis von Epoxidharzen, welche im Durchschnitt mehr als eine Epoxidgruppe im Molekül enthalten, und aminischen Härtungsmitteln für die Epoxidharze unter Mitverwendung von Hilfs- und Zusatzstoffen nach an sich bekannten Verfahren, welches dadurch gekennzeichnet ist, daß als Bindemittel härtbare Mischungen verwendet werden, welche bestehen aus:

A) mindestens einem Epoxidharz mit Epoxidwerten von 0,4 - 0,6 und

B) [1(2′Aminoethyl)-1,3-diaza-cyclopenten-2-yl-2-]-heptan-3 und/oder [1(2′Aminoethyl)-1,3-diaza-cyclopenten-2-yl-2)┤2,4,4-trimethylpentan-1

C) üblichen Füllstoffen, Pigmenten, Farbstoffen, Beschleunigern, Härtungsmitteln, Benetz- und Verlaufsmitteln, reaktiven Verdünnern.

In der industriellen Praxis werden auf vielen Gebieten noch überwiegend Behälter und Rohrleitungen aus metallischen Werkstoffen eingesetzt. Hierbei machen sich während der Einsatzdauer einige typische Metalleigenschaften negativ bemerkbar, insbesondere dort, wo wässrige oder sonstige korrosive Medien zum Einsatz kommen oder wo auf Gewichtseinsparung Wert gelegt werden muß.

Weiterhin müssen aufgrund der guten Wärmeleitfähigkeit der Metalle Behälter und Rohre überall dort aufwendig isoliert werden, wo außerhalb der Umgebungstemperatur gearbeitet werden muß.

Jährlich sind immense Summen aufzuwenden, um diese Nachteile durch entsprechende Maßnahmen auszugleichen.

Es sind daher seit geraumer Zeit verstärkte Anstrengungen unternommen worden, die positiven Eigenschafen von faserverstärkten Kunstharzen für dieses Gebiet nutzbar zu machen, wie geringes Gewicht, gute Chemikalienbeständigkeit - unter Umständen auch gegen Lösungsmittel -, Anpassungsfähigkeit hinsichtlich konstruktionstechnischer Anforderungen, wirtschaftliche Herstellung gegenüber anderen korrosionsbeständigen Werkstoffen wie Glas, Metall, Email, geringer Wartungs- u. Instandhaltungsaufwand.

Die Herstellung von Behältern und Rohren aus faserverstärkten Kunststoffen erfolgt im Wickelverfahren größtenteils maschinell. Die heutigen Fertigungstechniken erlauben aber nur die Herstellung relativ einfacher geometrischer Formen. Anschlußstücke für Ver- und Entsorgungsleitungen von Behältern, Ab- und Verzweigungen von Rohrleitungen sowie Herstellung von Rohrhilfsstücken müssen daher ausgespart werden und bleiben der manuellen Anfertigung vorbehalten.

Bei der heutigen Technik werden an den vorgesehenen Stellen der Körper die entsprechenden vorgefertigten Elemente anlaminiert.

Die Übergangsbereiche sind Schwachstellen, welche besondere Maßnahmen erfordern. Zur Erzielung der in der Praxis erforderlichen mechanischen Festigkeiten muß in diesen Bereichen der Anteil an Fasermaterial überdurchschnittlich erhöht werden, wodurch eine Reihe von besonderen Anforderungen an das Bindemittel weitergegeben werden, wie insbesondere gute Benetzungseigenschaften für die Fasern und Gewebe bei praxisgerechten Viskositäten und relativ lange offene Zeiten.

Die guten Benetzungseigenschaften sind ein wesentlicher Faktor, da bei den erforderlichen hohen Faseranteilen der Übergangsstellen die vollständige Durchtränkung aller, auch der Überlappungsbereiche, gewährleistet sein muß, um einen Abfall der mechanischen Werte auszuschließen. Hierbei muß gleichzeitig die Viskosität so angepaßt sein, daß beim Laminiervorgang kein Bindemittel ausgequetscht wird und auch vor oder während der Härtung im Gewebe gleichmäßig verteilt bleibt; d. h., daß sich das Bindemittel unter Schwerkrafteinwirkung nicht einseitig anreichert oder gar austritt.

Bei der überwiegend manuellen Herstellung muß die Topfzeit infolge der geringen Fertigungsgeschwindigkeiten entsprechend lang ausgelegt sein. Da bei großen Werkstücken naturgemäß große Mengen an Bindemittel benötigt werden, kann diese Forderung nicht durch Reduzierung der Ansatzgrößen überspielt werden.

Zur Erzielung einheitlicher und reproduzierbarer physikalisch-technischer Endwerte der gehärteten Produkte ist eine vorzeitige Gelierung unerwünscht. Die Gelierung darf also weder bei der Fertiung in einer Werkhalle noch bei Reparaturarbeiten vor Ort vor Fertigstellung der Arbeit eintreten. Hierbei muß die Einsatzsicherheit auch über einen den Jahreszeiten entsprechenden Temperaturbereich gegeben sein.

Die Resistenz von faserverstärkten Kunstharzen auf Basis von Epoxidharzen gegenüber Chemikalien ist bekannt.

Bei der Förderung von Wasser, insbesondere von heißem Wasser oder Dampf bis ca. 120 °C war jedoch nach relativ kurzer Zeit eine Verminderung des thermischen Eigenschaftsniveaus, z. B. (Torsionsschwingversuch DIN 53 445), zu verzeichnen.

Durch die Verminderung des thermischen Eigenschaftsniveaus werden die konstruktionstechnischen Eigenschaften der faserverstärkten Verbundwerkstoffe zum Teil soweit negativ beeinflußt, daß sie für die obengenannten Anwendungen nicht eingesetzt werden können.

Aus der Vielzahl der zur Verfügung stehenden Härtungsmittel konnte bisher nur das 1-Amino-3,3,5 Trimethyl-5-aminomethyl-cyclohexan (Isophorondiamin) die gestellten physikalischen Eigenschaftsanforderungen erfüllen.

Isophorondiamin ist aber ein relativ teurer Härter, welcher darüber hinaus nicht immer im erforderlichen Maße zur Verfügung steht und auch verarbeitungstechnisch einige Nachteile aufweist. Hier ist insbesondere hinzuweisen auf: kurze Verarbeitungszeit, hohe Härtungstemperatur, physiologische Probleme.

Es hat daher in der Vergangenheit nicht an Versuchen gemangelt, Alternativhärter zu entwickeln, welche dem Isophorondiamin vergleichbare Eigenschaften in den gehärteten Harzen erzeugen.

Die bislang vorgeschlagenen Imidazoline auf Basis von Umsetzungsprodukten aus geradkettigen monomeren Fettsäuren mit 2 bis 18 Kohlenstoffatomen, insbesondere mit 2 bis 5 Kohlenstoffatomen, brachten zwar eine physiologische Unbedenklichkeit, jedoch keine ausreichende Beständigkeit gegenüber heißem Wasser, was aus dem starken Abfall der HDT-Werte (Heat distortion temperature) nach Lagerung im kochenden Wasser ablesbar ist.

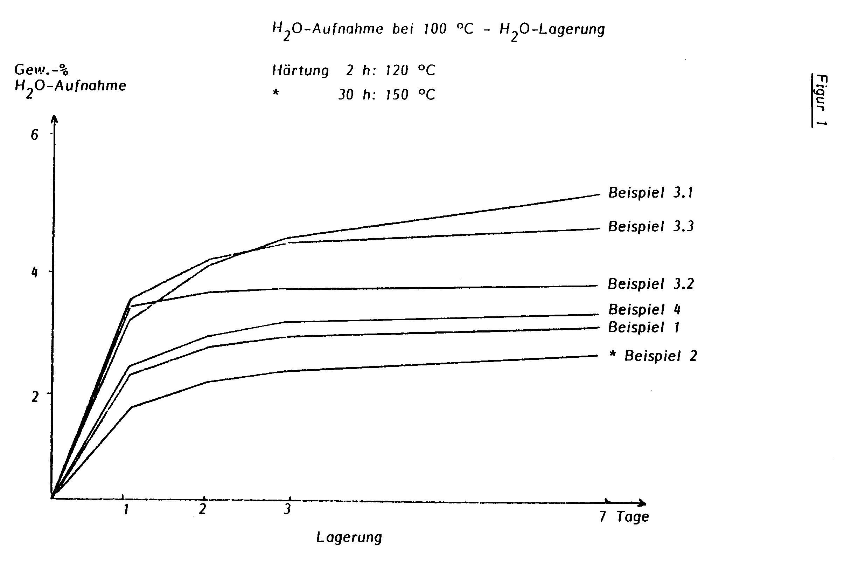

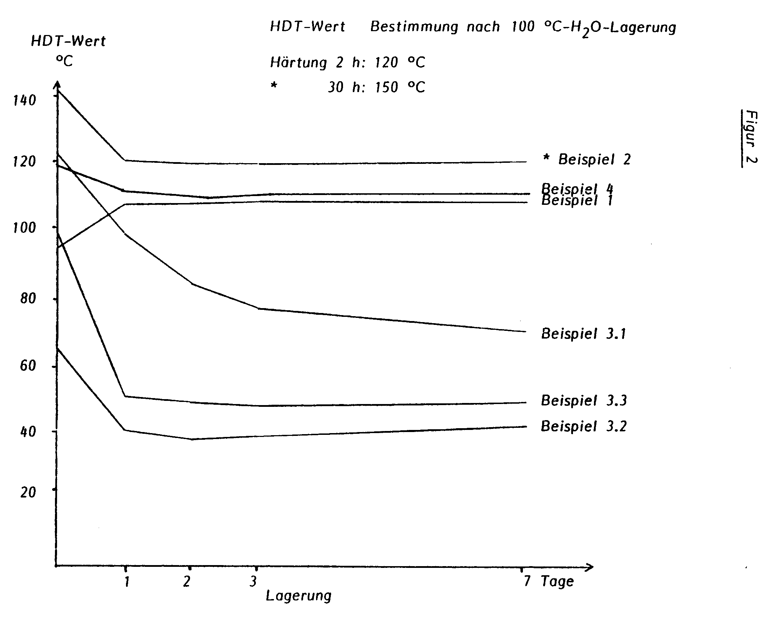

Die Erfahrungswerte aus der Praxis zeigten, daß nicht nur die Imidazoline aus kurzkettigen Monocarbonsäuren, sondern überraschenderweise auch die Imidazoline aus den hydrophoben langkettigen monomeren und sogar die verzweigten dimeren Fettsäuren größere Mengen an Wasser aufzunehmen vermögen. Entsprechend war der Abfall der HDT-Werte. Bei cycloaliphatischen Diaminen, wie Isophorondiamin war dies nicht der Fall (vergl. Figuren 1 und 2).

Nach allgemeiner Überzeugung waren Imidazoline auf Basis von Fettsäuren als Härter für Epoxidharze daher nicht geeignet für Produkte, welche später einer Wasserbelastung ausgesetzt waren.

Überraschenderweise wurde nun gefunden, daß die 2-Ethyl-hexansäure den gehärteten Epoxidharzen auf Basis von Bisphenol A und Bisphenol F Beständigkeit gegenüber einer Dauerbelastung von heißem Wasser oder Heißdampf bis ca. 120 °C verleiht, darüberhinaus sind auch die verarbeitungstechnischen Eigenschaften verbessert, insbesondere konnte die offene Zeit auf Zeiten verlängert werden, welche auch die manuelle Herstellung großer Werkstücke gestattet.

Gegenstand der Erfindung ist daher ein Verfahren zur Herstellung von faserverstärkten Wickelkörpern, insbesondere Rohrhilfsstücken, durch Tränken temperaturbeständiger Fasern mit Bindemitteln auf Basis von Epoxidharzen, welche im Durchschnitt mehr als eine Epoxidgruppe im Molekül enthalten, und aminischen Härtungsmitteln für die Epoxidharze unter Mitverwendung von Hilfs- und Zusatzstoffen nach an sich bekannten Verfahren, welches dadurch gekennzeichnet ist, daß als Bindemittel härtbare Mischungen verwendet werden, welche bestehen aus:

A) mindestens einem Epoxidharz mit Epoxidwerten von 0,2 - 0,6 und

B) [1(2′Aminoethyl)-1,3-diaza-cyclopenten-2-yl-2-]-heptan-3 und/oder [1(2′Aminoethyl)-1,3-diaza-cyclopenten-2-yl-2)]2,4,4-trimethylpentan-1

C) üblichen Füllstoffen, Pigmenten, Farbstoffen, Beschleunigern, Härtungsmitteln, Benetz- und Verlaufsmitteln, reaktiven Verdünnern.

Ein weiterer Gegenstand der Erfindung ist ein Verfahren, welches dadurch gekennzeichnet ist, daß als flüssiges Epoxidharz gemäß A) Bisphenol-A und/oder Bisphenol-F mit Epoxidwerten zwischen 0,45 bis 0,55 verwendet wird.

Die erfindungsgemäß mitverwendeten Epoxidharze sind Glycidylether mit zwei oder mehr Epoxygruppen pro Molekül wie vorzugsweise die Glycidylether auf Basis von ein- oder mehrwertigen Phenolen. Erfindungsgemäß bevorzugt werden Glycidylether von 2,2-Bis(4-hydroxyphenyl)-propan (Bisphenol A) mit Epoxidwerten von 0,4 - 0,6, insbesondere die bei Raumtemperatur flüssigen Verbindungen mit Epoxidwerten um 0,45 bis 0,55. Daneben haben sich auch die Glycidylether auf Basis von Bisphenol F und die Novolake als vorteilhaft erwiesen.

Das [1(2′Aminoethyl)-1,3-diaza-cyclopenten-2-yl-2-]-heptan-3 wird hergestellt durch Umsetzung von 2-Ethyl-hexansäure mit Diethylentriamin im Molverhältnis von mindestens 1 : 1. Das [1(2′Aminoethyl)-1,3-diaza-cyclopenten-2-yl-2)]2,4,4-trimethylpentan-1 wird hergestellt durch Umsetzung von 3,5,5-Trimethylhxansäure mit Diethylentriamin im Molverhältnis von mindestens 1 : 1. Sie werden in der Regel in Mengen von 10 - 20 Gew.-Teilen, vorzugsweise 12 - 18, insbesondere 15, Gew.-Teilen pro 100 Gew.-Teile Epoxidharz mitverwendet.

Falls gewünscht, können zur Modifizierung der Verarbeitungs- und Härtungseigenschaften auch die auf diesem Gebiet üblichen und allgemein bekannten Modifizierungsmittel wie übliche Füll- und/oder Versteifungsmaterialien, Pigmente, Farbstoffe, Beschleuniger, Benetz- und Verlaufsmittel, reaktive Verdünner, Härtungsmittel mitverwendet werden. Als Verstärkungsmaterialien werden die üblichen Glasfasern bevor-

3

zugt eingesetzt.

Als erfindungsgemäß mitverwendbare Glycidylether werden solche auf Basis von alicyclischen Alkoholen wie 1,4-Dimethylolcyclohexan und aliphatischen Alkoholen, insbesondere 2- oder 3-wertigen aliphatischen Alkoholen mit 4 - 8 C-Atomen wie Butandiole, Hexandiole, Oktandiole, Glycerin, welche durch Addition von Ethylenoxid oder Propylenoxid verlängert sein können, bevorzugt.

Die Menge der reaktiven Verdünner liegt in der Regel zwischen 5 - 10, vorzugsweise 6 - 8 Gew.-%, bezogen auf Epoxidharz gemäß A).

Als übliche Härtungsmittel werden insbesondere die cycloaliphatischen Amine wie beispielsweise Isophorondiamin, 1,2-Diaminocyclohexan, 4,4′-Diamino-3,3′-dimethyl-dicylohexylmethan, Bis{1-(2′aminoethyl)-1,3-diaza-cyclopenten-2-yl-2}heptan-1,7,Bis{1(2′aminoethyl)-1,3-diaza-cyclopenten-2-yl-2}oktan-1,8 mitverwendet.

## Beschreibung des Laminier-Verfahrens

Die Herstellung eines (nicht) rechtwinkligen Abzweigstückes von einem Hauptrohr größeren Durchmessers (Hauptrohr) geschieht, wie bereits erwähnt, unter Zuhilfenahme maschinell vorgefertigter Rohrstücke. Im ersten Schritt wird in das ausgehärtete Hauptrohr eine dem Durchmesser des Abzweigrohres entsprechende Öffnung geschnitten. Im zweiten Schritt erfolgt eine Abschrägung des einen Endes des ebenfalls bereits ausgehärteten Abzweigrohres in dem gewünschten Abzweigwinkel und im dritten Schritt werden beide Rohre nach an sich bekannten Verfahren aneinander laminiert. Dazu werden sie zunächst mittels einer Hilfseinrichtung gegeneinander fixiert, und anschließend wird über dem Verbindungsbereich manuell ein Naßlaminat aufgebaut.

Für einen lunkerfreien Laminataufbau und eine ausreichend hohe Festigkeit der Rohrverbindung ist es wichtig, daß das verwendete Harzsystem die eingesetzten Verbindungsmaterialien optimal benetzt und auch auf den Oberflächen der zu verbindenden Rohrelemente gut haftet.

Die Forderung nach optimalen Benetzungseigenschaften bedingt jedoch die Verwendung eines bereits bei Raumtemperatur sehr niedrigviskosen Laminiersystems, da eine Viskositätsanpassung durch Aufheizen des Systems wegen der manuellen Arbeitsweise kaum möglich ist. Außerdem darf das Material nicht schon während des Laminierens gelieren, da hierdurch eine deutliche Minderung der Haftung zwischen den einzelnen Laminatlagen hervorgerufen werden kann. Weiterhin erleichtert eine lange Gelierungszeit des Systems den Fertigungsvorgang erheblich, da auch bei großflächigen Verbindungsbereichen mit nur einem Materialansatz gearbeitet werden kann, wodurch die Gefahr von Misch- und Dosierfehlern deutlich herabgesetzt wird.

Nach Beendigung des Laminataufbaus erfolgt eine thermische Aushärtung der Verbindungsstelle. Dieser Härtungsvorgang sollte jedoch auf möglichst geringem Temperaturniveau vorgenommen werden können, um zum einen die thermischen Spannungen im Bauteil so gering wie möglich zu halten und um zum anderen die Härtung gegebenenfalls auch auf der Baustelle vornehmen zu können.

Im Hinblick auf ein gutes Eigenschaftsniveau des Gesamtbauteils sollten möglichst die Eigenschaften der Verbindungsstelle mit denen der verbundenen Rohrstücke übereinstimmen, um materialbedingte Spannungsspitzen im Bauteil auszuschließen. Da verstärkungsseitig der Einsatz des gleichen Fasermaterials für Rohrstücke und Verbindungsstelle meist keine Schwierigkeiten bereitet, ist die obengenannte Forderung immer dann erfüllt, wenn auch auf der Matrixseite ähnliche Eigenschaften erreicht werden.

## Beispiel 1

38,88 kg 2-Ethylhexansäure
27,81 kg Diethylentriamin und
0,34 kg p-Toluolsulfonsäure
werden unter Stickstoff chargiert. Unter Rühren wird das Gemisch auf ca. 160 - 170 °C aufgeheizt; bei dieser Temperatur beginnt die Kondensation. Anschließend wird die Temperatur langsam auf maximal 280 °C erhöht, wobei mit einem Aminkühler erreicht wird, daß nur Wasser und nicht Diethylentriamin abdestilliert. Danach wird langsam Vakuum bis zu ca. 800 mbar angelegt.

Das Reaktionsgemisch wird so lange bei 280 °C/ca. 800 mbar gehalten, bis die errechnete Kondensatmenge erreicht ist und der Imidazolingehalt - ermittelt durch Infrarotspektroskopie - mehr als 80 % beträgt.

Das filtrierte Produkt hat folgende Kennzahlen:

4

EP 0 375 991 B1

| Aminzahl: | ca. 450 |
|---|---|
| Viskosität: | ca. 500 m Pa.s/25 °C |
| Imidazolingehalt: | ca. 80 % |

Beispiel 2 (Vergleich)

| Handelsübliches Isophorondiamin | |
|---|---|
| Aminzahl: | 660 |
| Viskosität: | 15 m Pa.s/25 °C |

Beispiel 3 (Vergleich)

Wie Beispiel 1, jedoch kommt anstelle von 2-Ethylhexansäure die äquivalente Menge 3.1 Propionsäure, 3.2 Tallölfettsäure, 3.3 Dimere Fettsäure (96 % Dimeranteil) zum Einsatz. Das Produkt hat folgende Kennzahlen:

| 3.1 | Aminzahl | 700 |
|---|---|---|
| | Viskosität | 20 mPa.s/25 °C |
| | Imidazolingehalt | 80 % |
| 3.2 | Aminzahl | 260 |
| | Viskosität | 200 mPa.s/25 °C |
| | Imidazolingehalt | 91 % |
| 3.3 | Aminzahl | 300 |
| | Viskosität | 5000 mPa.s/25 °C |

Beispiel 4

Imidazolin aus 3,3,5-Trimethylhexansäure (Isononansäure) und Diethylentriamin

316 g Isononansäure
206 g Diethylentriamin und
0,5 g p-Toluolsulfonsäure

werden unter Stickstoff chargiert. Unter Rühren wird das Gemisch auf ca. 160 - 170 °C aufgeheizt; bei diser Temperatur beginnt die Kondensation. Anschließend wird die Temperatur langsam auf maximal 260 °C erhöht, wobei mit einem Aminkühler erreicht wird, daß nur Wasser und nicht Diethylentriamin abdestilliert. Da unter diesen Bedingungen die theoretische Wassermenge nicht abdestilliert, wird langsam Vakuum bis zu ca. 100 mbar angelegt.

Das Reaktionsgemisch wird solange bei 260 °C/ca. 100 mbar gehalten, bis die errechnete Kondensatmenge erreicht ist und der Imidazolingehalt - ermittelt durch Infrarotspektroskopie - mehr als 80 % beträgt.

Das filtrierte Produkt hat folgende Kennzahlen:

| Aminzahl | 437 |
|---|---|
| Viskosität | 105 mPa.s/25 °C |
| Imidazolingehalt | 88 % |

5

Versuchsdurchführung

Zur Ermittlung des mechanischen Eigenschaftsniveaus werden 15 Gew.-Teile des wie für Beispiel 1, Beispiel 3 und Beispiel 4 beschriebenen Produkts und 25 Gew.-Teile des für Beispiel 2 beschriebenen Produktes mit 100 Gew.-Teilen eines niedrigviskosen Epoxidharzes auf Bisphenol-A-Basis (Epoxid-Wert 0,54) vermischt und in einem Stahlwerkzeug in 2 Stunden bei 120 °C zu 4 mm dicken ebenen Formteilen ausgehärtet.

Aus diesen Formteilen werden dann durch Sägen bzw. Fräsen Probekörper entnommen, an denen unter Einhaltung der jeweiligen Prüfnormen die in nachstehender Tabelle 1 aufgeführten Eigenschaftswerte ermittelt sind.

Die bei den verschiedenen Prüfungen benutzten Prüfkörperabmessungen sind:

3-Pkt.-Biegeversuch: 80 x 10 x 4 mm$^3$

Zugversuch: Schalterhub Nr. 3 nach DIN 53 455

HDT: 120 x 10 x 4 mm$^3$

## Tabelle 1

Eigenschaften verschiedener Bindemittelsysteme nach Härtung von 2 h bei 120 °C

| Eigenschaft | Einheit | Beispiel 1 | Beispiel 2 | Beispiel 3.1 | Beispiel 4 |
|---|---|---|---|---|---|
| Tecam-Gelierungszeit für 250 g bei 23 °C | min | 1200 | 115 | 400 | 760 |
| Biegefestigkeit (DIN 53452) | N/mm² | 102 | 102 | 110 | 60 |
| Zugfestigkeit (DIN 53455) | N/mm² | 67 | 45,3 | 75 | 65 |
| Dehnung (DIN 53455) | % | 3,1 | 1,9 | 3,5 | 3,5 |
| Heat dist. temperature (DIN 53461) | °C | 108 | 123 | 120 | 123 |
| Übergangstemperatur (DIN 53445) | °C | 145 | 158 | 145 | 157 |

EP 0 375 991 B1

Tabelle 2

| HDT-Werte verschiedener Bindemittelsysteme nach Lagerung in kochendem Wasser | | | | | |
|---|---|---|---|---|---|
| | Lagerzeit in kochendem Wasser | | | | |
| | 0-Wert | 1 Tag | 2 Tage | 3 Tage | 7 Tage |
| Beispiel 1 | 108 | 108 | 108 | 109 | 109 |
| Beispiel 2 | 123 | 118 | 118 | 117 | 117 |
| Beispiel 3.1 | 120 | 99 | 84 | 77 | 70 |
| Beispiel 4 | 123 | 111 | 110 | 110 | 111 |

**Patentansprüche**

1. Verfahren zur Herstellung von faserverstärkten Wickelkörpern, insbesondere Rohrhilfsstücken, durch Tränken temperaturbeständiger Fasern mit Bindemitteln auf Basis von Epoxidharzen, welche im Durchschnitt mehr als eine Epoxidgruppe im Molekül enthalten, und aminischen Härtungsmitteln für die Epoxidharze unter Mitverwendung von Hilfs- und Zusatzstoffen nach an sich bekannten Verfahren, dadurch gekennzeichnet, daß als Bindemittel härtbare Mischungen verwendet werden, welche bestehen aus:

    A) mindestens einem Epoxidharz mit Epoxidwerten von 0,4 - 0,6 und

    B) [1(2′Aminoethyl)-1,3-diaza-cyclopenten-2-yl-2-]-heptan-3 und/oder [1(2′Aminoethyl)-1,3-diaza-cyclopenten-2-yl-2)-]-2,4,4-trimethylpentan-1

    C) üblichen Füllstoffen, Pigmenten, Farbstoffen, Beschleunigern, Härtungsmitteln, Benetz- und Verlaufsmitteln, reaktiven Verdünnern.

2. Verfahren gemäß Anspruch 1, dadurch gekennzeichnet, daß als flüssiges Epoxidharz gemäß A) Bisphenol A und/oder Bisphenol F mit Epoxidwerten zwischen 0,45 bis 0,55 verwendet wird.

3. Verfahren gemäß den Ansprüchen 1 und 2, dadurch gekennzeichnet, daß das Bindemittel aus den Komponenten A) und B) bei Verarbeitungstemperatur flüssig ist.

4. Verfahren gemäß den Ansprüchen 1 - 3, dadurch gekennzeichnet, daß das [1(2′Aminoethyl)-1,3-diaza-cyclopenten-2-yl-2-]-heptan-3 und/oder das [1(2′Aminoethyl)-1,3-diaza-cyclopenten-2-yl-2)-]-2,4,4-trimethylpentan-1 in Mengen von 10 - 20 Gew.-Teilen, max. 20 Gew.-Teilen pro 100 Gew.-Teile Epoxidharz mitverwendet wird.

5. Verfahren gemäß den Ansprüchen 1 bis 3, dadurch gekennzeichnet, daß als reaktive Verdünner Glycidylether auf Basis von aliphatischen oder alicyclischen Polyolen mit 4 - 8 C-Atomen mitverwendet werden.

6. Verfahren gemäß den Ansprüchen 1 - 5, dadurch gekennzeichnet, daß als übliche Härtungsmittel gemäß C) cycloaliphatische Amine mitverwendet werden.

7. Verwendung von härtbaren Mischungen aus

    A) mindestens einem Epoxidharz mit Epoxidwerten von 0,4 - 0,6 und

    B) [1(2′Aminoethyl)-1,3-diaza-cyclopenten-2-yl-2-]-heptan-3 und/oder [1(2′Aminoethyl)-1,3-diaza-cyclopenten-2-yl-2)-]-2,4,4-trimethylpentan-1

    C) üblichen Füllstoffen, Pigmenten, Farbstoffen, Beschleunigern, Härtungsmitteln, Benetz- und Verlaufsmitteln, reaktiven Verdünnern.

    zur Herstellung von Anschlußstücken für Ver- und Entsorgungsleitungen von Behältern, Ab- und Verzweigungen von Rohrleitungen, Rohrhilfsstücken.

**Claims**

1. Process for the manufacture of fibre-reinforced wound members, especially pipe sections, by impregnating temperature-resistant fibres with binders based on epoxy resins containing on average more than one epoxy group in the molecule, and amine curing agents for the epoxy resins, with the concomitant use of auxiliaries and additives according to processes known per se, characterised in that there are used as binders curable mixtures consisting of:

A) at least one epoxy resin having epoxy values of 0.4 - 0.6 and

B) 3-[1(2'aminoethyl)-1,3-diaza-2-cyclopenten-2-yl]heptane and/or 1-[1(2'aminoethyl)-1,3-diaza-2-cyclopenten-2-yl)]-2,4,4-trimethylpentane and

C) customary fillers, pigments, dyestuffs, accelerators, curing agents, wetting agents and flow agents, reactive diluents.

2. Process according to claim 1, characterised in that bisphenol A and/or bisphenol F having epoxy values of from 0.45 to 0.55 is/are used as liquid epoxy resin according to A).

3. Process according to claims 1 and 2, characterised in that the binder comprising components A) and B) is liquid at the processing temperature.

4. Process according to claims 1 to 3, characterised in that the 3-[1(2'aminoethyl)-1,3-diaza-2-cyclopenten-2-yl]-heptane and/or the 1-[1(2'aminoethyl)-1,3-diaza-2-cyclopenten-2-yl)]-2,4,4-trimethylpentane is/are used concomitantly in amounts of from 10 to 20 parts by weight, max. 20 parts by weight, per 100 parts by weight of epoxy resin.

5. Process according to claims 1 to 3, characterised in that there are used concomitantly as reactive diluents glycidyl ethers based on aliphatic or alicyclic polyols having from 4 to 8 carbon atoms.

6. Process according to claims 1 to 5, characterised in that cycloaliphatic amines are used concomitantly as customary curing agents according to C).

7. Use of curable mixtures consisting of

A) at least one epoxy resin having epoxy values of from 0.4 to 0.6 and

B) 3-[1(2'aminoethyl)-1,3-diaza-2-cyclopenten-2-yl]heptane and/or 1-[1(2'aminoethyl)-1,3-diaza-2-cyclopenten-2-yl)]-2,4,4-trimethylpentane, and

C) customary fillers, pigments, dyestuffs, accelerators, curing agents, wetting agents and flow agents, reactive diluents

for the preparation of connecting pieces for supply and discharge lines for containers, branches and junctions of pipes, and pipe sections.

**Revendications**

1. Procédé pour fabriquer des corps de bobinage renforcés de fibres, particulièrement des pièces auxiliaires de tuyaux, par imprégnation de fibres résistantes à la chaleur par des liants à base de résines époxydes qui contiennent en moyenne plus d'un groupe époxy par molécule, et de durcisseurs pour les résines époxydes, avec emploi simultané de matières auxiliaires et d'additifs, selon des méthodes connues, procédé caractérisé en ce qu'on utilise, comme liant, des mélanges durcissables constitués de :

A) d'au moins une résine époxyde ayant des indices d'époxyde de 0,4 à 0,6 et

B) de 3-[1-(2'-aminoéthyl)-1,3-diaza-cyclopentène-2-yl-2]heptane et/ou de 1-[1-(2'-aminoéthyl)-1,3-diazacyclopentène-2-yl-2]-2,4,4-triméthylpentane,

C) de charges, pigments, colorants, accélérateurs, durcisseurs, agents mouillants et fluidifiants, diluants réactifs, pris parmis ceux qui sont d'un usage courant.

2. Procédé selon la revendication 1, caractérisé en ce qu'on utilise comme résine époxyde liquide selon A), un bisphénol A et/ou un bisphénol F ayant des indices d'époxyde compris entre 0,45 et 0,55.

3. Procédé selon les revendications 1 et 2, caractérisé en ce que le liant constitué des composants A) et B) est liquide à la température de mise en oeuvre.

4. Procédé selon les revendications 1 à 3, caractérisé en ce qu'on utilise le 3-[1-(2'-aminoéthyl)-1,3-diazacyclopentène-2-yl-2]-heptane et/ou le 1-[1-(2'-aminoéthyl)-1,3-diaza-cyclopentène-2-yl-2)-]-2,4,4-triméthylpentane en des quantités de 10 à 20 parties pondérales, d'au plus 20 parties en poids, pour 100 parties en poids de résine époxyde.

5.   Procédé selon les revendications de 1 à 3, caractérisé en ce qu'on utilise, comme diluants réactifs, des éthers glycidyliques dérivant de polyols aliphatiques ou alicycliques comportant de 4 à 8 atomes de carbone.

6.   Procédé selon les revendications de 1 à 5, caractérisé en ce qu'on utilise, comme durcisseurs habituels selon C) des amines cycloaliphatiques.

7.   Application de mélanges durcissables constitués :
A) d'au moins une résine époxyde ayant des indices d'époxyde de 0,4 à 0,6 et
B) de 3-[1-(2'-aminoéthyl)-1,3-diaza-cyclopentène-2-yl-2-]-heptane et/ou de 1-[1-(2'-aminoéthyl)-1,3-diazacyclopentène-2-yl-2)]-2,4,4-triméthylpentane,
C) de charges, pigments, colorants, accélérateurs, durcisseurs, agents mouillants et fluidifiants, diluants réactifs, pris parmi ceux qui sont habituellement utilisés,
pour la fabrication de pièces d'assemblage raccord pour conduites d'alimentation et d'évacuation de récipients, de branchements et de ramifications de canalisations rigides, de pièces auxiliaires de tuyaux.

EP 0 375 991 B1

Figur 1

H₂O-Aufnahme bei 100 °C – H₂O-Lagerung

Härtung  2 h: 120 °C
*        30 h: 150 °C

Beispiel 3.1
Beispiel 3.3
Beispiel 3.2
Beispiel 4
Beispiel 1
* Beispiel 2

Gew.-%
H₂O-Aufnahme

6

4

2

1    2    3                Lagerung              7 Tage

11

HDT-Wert    Bestimmung nach 100 °C-$H_2O$-Lagerung

Härtung 2 h: 120 °C
*          30 h: 150 °C

HDT-Wert
°C

140

120                                                    * Beispiel 2

                                                       Beispiel 4
                                                       Beispiel 1
100

80
                                                       Beispiel 3.1

60

                                                       Beispiel 3.3
40                                                     Beispiel 3.2

20

        1      2      3                    7  Tage
                   Lagerung

Figur 2

EP 0 375 991 B1